(19) Europäisches Patentamt
European Patent Office
Office européen des brevets

(11) **EP 2 121 136 B1**

(12) **FASCICULE DE BREVET EUROPEEN**

(45) Date de publication et mention
de la délivrance du brevet:
**28.11.2012 Bulletin 2012/48**

(21) Numéro de dépôt: **08762136.3**

(22) Date de dépôt: **20.02.2008**

(51) Int Cl.:
*A61N 7/02* *(2006.01)*    *G01S 7/52* *(2006.01)*

(86) Numéro de dépôt international:
**PCT/FR2008/050290**

(87) Numéro de publication internationale:
**WO 2008/113940 (25.09.2008 Gazette 2008/39)**

(54) **PROCEDE D'OPTIMISATION DE LA FOCALISATION D'ONDES AU TRAVERS D'UN ELEMENT INTRODUCTEUR D'ABERRATIONS**

VERFAHREN ZUR OPTIMIERUNG DER WELLENFOKALISIERUNG DURCH EIN ABERRATIONSEINSATZELEMENT

METHOD FOR OPTIMISING WAVE FOCALISATION THROUGH AN ABERRATION INSERTION MEMBER

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **21.02.2007 FR 0701235**

(43) Date de publication de la demande:
**25.11.2009 Bulletin 2009/48**

(73) Titulaires:
• **Super Sonic Imagine**
  **13857 Aix En Provence Cedex (FR)**
• **Centre National de la Recherche Scientifique (CNRS)**
  **75794 Paris Cedex 16 (FR)**

(72) Inventeurs:
• **PERNOT, Mathieu**
  **75004 Paris (FR)**

• **FINK, Mathias**
  **92190 Meudon (FR)**
• **TANTER, Mickaël**
  **92220 Bagneux (FR)**
• **MONTALDO, Gabriel**
  **75009 Paris (FR)**
• **AUBRY, Jean-François**
  **92340 Bourg La Reine (FR)**
• **SINKUS, Ralph**
  **75018 Paris (FR)**

(74) Mandataire: **Domange, Maxime et al**
**Cabinet Beau de Lomenie**
**232, avenue du Prado**
**13295 Marseille Cedex 08 (FR)**

(56) Documents cités:
EP-A2- 0 320 303    US-A- 5 357 962
US-A- 5 423 318    US-A1- 2004 122 323

**Description**

Arrière-plan de l'invention

La présente invention se rapporte au domaine général de la focalisation d'ondes au sein d'un milieu. Plus précisément, l'invention concerne les modalités d'imagerie nécessitant la focalisation d'ondes à travers des milieux fortement hétérogènes. Les applications de ce type d'imagerie sont multiples. Il s'agit notamment de l'acoustique sous marine, des télécommunications, de la géophysique, des contrôles non destructifs de matériaux, de la médecine etc. Dans ce dernier domaine, les ondes focalisées sont utilisées en particulier en imagerie et en thérapie, notamment les ondes ultrasonores focalisées.

Les aberrations introduites par les milieux hétérogènes sont un problème dans chacune de ces applications.

En effet, dès qu'une intensité importante est véhiculée par les ondes ou qu'il est très important qu'uniquement une zone délimitée soit soumise aux ondes, les aberrations sont très pénalisantes et peuvent même empêcher l'utilisation d'ondes focalisées dans certaines applications à cause de l'impossibilité d'assurer une focalisation correcte. Certaines applications, par exemple dans le domaine de la thérapie, requièrent en effet que la focalisation soit très précise afin de ne pas élargir la zone d'action des ondes focalisées.

Actuellement, la précision requise dans de telles applications est rendue accessible au prix d'une grande complexité de mise en oeuvre, et, dans le cas de la thérapie, au prix d'une chirurgie invasive.

En effet, afin de pouvoir assurer une focalisation correcte d'ondes ultrasonores de haute intensité dans le cadre de la thérapie du cerveau, une première solution, décrite dans le document FR 2 843 874, consiste à exploiter des résultats d'imagerie du cerveau du patient obtenue par balayage tomographique (scan CT pour Computed Tomography en anglais) réalisé préalablement au traitement. Les informations sur la structure en trois dimensions de l'os du crâne sont alors utilisées pour simuler les aberrations et corriger les signaux émis pendant le traitement. Ces simulations numériques prennent généralement un temps important incompatible avec un travail en temps réel.

Ensuite, le patient doit être repositionné dans un appareil d'imagerie à résonance magnétique pour permettre un contrôle du traitement en temps réel, généralement par imagerie de l'élévation de température.

Il est alors nécessaire que le patient soit repositionné par rapport à un référentiel identique à celui utilisé lors du scan CT afin que les structures soient pareillement disposées. Cela implique une procédure de recalage généralement complexe. Des cadres de stéréotaxie vissés sur la tête du patient peuvent notamment être utilisés.

Une seconde solution est basée sur le retournement temporel des ondes. Elle consiste à implanter une minuscule sonde ultrasonore émettrice d'ondes ultrasonores sur un instrument chirurgical utilisé au cours d'une biopsie. Lors d'un prélèvement de tissu, la sonde émet, au voisinage de la tumeur, des ondes ultrasonores qui sont captées et enregistrées à leur sortie de la boite crânienne par un réseau de transducteurs ultrasonores.

Si la biopsie révèle la nécessité de traiter, les signaux enregistrés sont alors retournés temporellement. Lors de leur réémission, les signaux retournés focalisent alors automatiquement sur la zone où a eu lieu la biopsie c'est-à-dire au niveau de la zone tumorale. Il est néanmoins nécessaire que le patient soit recalé correctement par rapport au réseau de transducteurs ce qui peut se révéler être de nouveau une opération délicate.

Ensuite, en pratique, pour réaliser le traitement, le faisceau est déplacé électroniquement autour du point focal initial pour traiter l'ensemble de la tumeur point par point.

Cette dernière solution est invasive et comporte donc tous les inconvénients inhérents aux procédés invasifs.

US2004/012323 divulgue un procédé pour la déduction d'une distribution de phases et/ou d'amplitudes permettant une amélioration de la focalisation d'ondes au travers d'un élément introducteur d'aberrations. Le procédé décrit dans ce document utilise l'acquisition préalable d'une image pour déterminer les frontières entre les différents types de tissus ainsi que les différents types de tissus traversés par les ondes pour déterminer des facteurs de correction compensant la réfraction sur les zones inter faciales et compensant les variations de vitesse du son dans les différents types de tissus.

US5590657 divulgue également un procédé de focalisation d'ondes acoustiques en une zone souhaitée. Le procédé divulgué dans US5590657 prend une image de la zone d'intérêt, localise la zone de traitement par rapport à la zone souhaitée et corrige la focalisation en conséquence.

L'utilisation d'ondes focalisées dans le cadre médical, en particulier l'utilisation des ondes ultrasonores focalisées de haute intensité, n'est donc actuellement pas privilégiée dès lors que des éléments introducteurs d'aberrations se trouvent sur le trajet des ondes. Pourtant, les ondes ultrasonores présentent des atouts majeurs parmi lesquels la possibilité d'intervenir à tout moment en cas de récidive et la possibilité d'intervenir sur les enfants pour lesquels les options thérapeutiques sont plus limitées que pour l'adulte.

Il y a aussi lieu de souligner que l'utilisation d'ondes ultrasonores à haute intensité est une intervention dépourvue d'irradiation réalisée par seule élévation locale de température.

Tous les moyens qui permettent l'extension du domaine d'utilisation des ondes ultrasonores, et plus généralement des ondes non irradiantes, sont donc dignes du plus grand intérêt. En particulier, les moyens permettant d'éliminer l'influence des aberrations comptent parmi ces moyens.

Objet et résumé de l'invention

La présente invention a donc pour but principal de permettre de réaliser des focalisations correctes d'ondes même en présence d'un élément aberrateur tout en évitant les inconvénients présentés par les deux solutions existantes, à savoir la complexité d'un recalage entre deux modalités d'imagerie accompagné de risques d'erreurs et, dans le cas du guidage par biopsie , le caractère invasif.

L'invention propose pour cela un procédé d'optimisation de la focalisation d'ondes dans une zone d'intérêt d'un milieu, les ondes étant émises par un réseau de N sources vers le milieu au travers d'un élément introducteur d'aberrations introduisant un décalage de phase initialement indéterminé $\varphi_n$ ($1 \leq n \leq N$), le procédé utilisant M-1 modifications successives de l'onde émise entrainant chacune une perturbation, les M perturbations sont mesurées, ces mesures étant utilisées pour déduire des caractéristiques de focalisation optimale, et comprenant les étapes suivantes :

a) émission, par un réseau de N sources, d'ondes $A_n . e^{j\alpha_n}$ (avec pour notation j nombre complexe vérifiant $j^2 = -1$) présentant une distribution spatiale de phase $\alpha_n$ et d'amplitude $A_n$ ($1 \leq n \leq N$) et se propageant vers la zone d'intérêt dans le milieu,

b) M-1 (M>1) modification(s) de la distribution spatiale de phase $\alpha_n$ et/ou d'amplitude $A_n$ ($1 \leq n < N$) des ondes émises simultanément par une pluralité de sources du réseau, chaque modification correspondant à des modifications simultanées des phases et/ou amplitudes sur cette pluralité de sources du réseau et engendrant chacune une étape a) d'émission,

c) mesure d'au moins une perturbation $I_m$ ($1 \leq m \leq M$) induite par les ondes dans la zone d'intérêt à chaque modification m ($1 \leq m \leq M$) de la distribution des phases $\alpha_n$ et/ou d'amplitudes $A_n$ ,

d) déduction, à partir des mesures de perturbation $I_m$, d'une distribution optimale de phase d'émission $\alpha_{n_{opt}}$ et/ou une distribution d'amplitude $A_{n_{opt}}$ maximisant la perturbation induite dans la zone d'intérêt.

Un tel procédé propose une adaptation de la focalisation en utilisant la mesure d'au moins une perturbation induite par l'onde focalisée dans la zone focale théorique pour optimiser les décalages de phase sur l'ensemble des transducteurs générant l'onde focalisée ou, au moins sur une pluralité d'entre eux. La perturbation est générée à distance dans la zone d'intérêt par le réseau d'émetteurs.

Par le terme « perturbation », on entend une modification réversible ou irréversible de certains paramètres physiques du milieu résultant de interaction entre l'onde et le milieu liée à la densité d'énergie ou de puissance moyenne transportée par l'onde dans la zone d'intérêt ou une densité d'énergie de l'onde incidente dans la zone d'intérêt.

Une conséquence importante de cette définition restrictive de la perturbation est que l'amplitude de la perturbation est liée uniquement à l'amplitude de l'onde ou à son énergie dans la zone d'intérêt et non à sa phase à un instant donné. La perturbation peut-être par exemple une augmentation de température locale résultant de l'absorption de l'onde par le milieu, un déplacement ou une déformation locale du milieu résultant de la force de radiation exercée par l'onde, ou encore l'apparition d'une bulle de cavitation, une énergie rétrodiffusée par la zone d'intérêt, une densité d'énergie acoustique, ultrasonore, électromagnétique ou optique dans la zone d'intérêt.

La mesure de la perturbation est rendue possible par le fait que les perturbations sont induites dans le milieu par plusieurs sources du réseau en même temps et non pas successivement par une seule source. Ceci assure que l'énergie émise dans le milieu est suffisante pour induire une perturbation détectable par le système de mesure et que les modifications, effectuées sur une pluralité de sources simultanément, génèrent des perturbations différentiables. La focalisation optimale est déduite des émissions successives réalisées à chaque fois avec plusieurs sources du réseau après modifications successives de la distribution de phase et/ou d'amplitude.

On entend par les termes « zone d'intérêt » une zone du tissu dans laquelle on souhaite focaliser les ondes afin d'y atteindre spatialement un maximum d'énergie. Typiquement, par la focalisation d'ondes, on cherchera à atteindre un pic d'énergie sur une zone d'intérêt limitée à une extension de quelques centaines de $\mu$m en profondeur et latéralement dans le domaine des ultrasons. De manière plus générale, une zone d'intérêt pour une modalité donnée utilisant des ondes présente une dimension voisine de la longueur d'onde utilisée.

L'invention est particulièrement intéressante dans le cadre de la thérapie du cerveau où il est nécessaire de repérer très précisément la zone à nécroser, typiquement une tumeur, et de ne pas sortir de cette zone afin de ne pas causer de dommages aux tissus sains. Les traitements par faisceaux ultrasonores focalisés de forte intensité sont donc particulièrement concernés par l'invention.

Dans ce cadre, avec l'aide de l'invention, la correction de focalisation peut être réalisée juste avant le traitement avec un seul et même réseau de transducteurs servant pour l'adaptation de focalisation, le traitement, voire aussi l'imagerie.

En outre, permettant une intervention sans trépanation ni introduction d'instruments chirurgicaux, l'invention offre une qualité et un confort d'intervention jusque là inaccessible.

En particulier, l'invention rend aisée les thérapies non invasives des lésions cérébrales. Comme ces thérapies sont avantageuses des points de vue des coûts et des risques, l'invention répond donc aux enjeux socio-économiques actuels dans le domaine des interventions neurochirurgicales.

Selon une caractéristique additionnelle avantageuse, le procédé selon l'invention comprend en outre une étape c') de mesure d'au moins une perturbation $I_{me}$ en un point situé hors de la zone d'intérêt [6] à chaque modification m ($1 \leq m \leq M$) de la distribution des phases $\alpha_n$ et/ou d'amplitudes $A_n$, l'étape d) étant alors une étape de déduction, à partir des mesures de perturbation $I_m$ et des mesures de perturbation $I_{me}$, d'une distribution optimale de phase d'émission $\alpha_{n_{opt}}$ et/ou une distribution d'amplitude $A_{n_{opt}}$ maximisant la perturbation $I_m$ induite dans la zone d'intérêt [6] et minimisant la ou les perturbations $I_{me}$ hors de la zone d'intérêt.

Cette caractéristique additionnelle avantageuse permet de concentrer d'autant plus l'énergie envoyée dans le milieu au niveau de la zone d'intérêt et non, à l'extérieur de celle-ci. Cette caractéristique permet donc de contrôler l'étendue de la perturbation idéalement limitée à la zone d'intérêt.

Plus généralement, à chaque modification, plusieurs mesures de la perturbation peuvent être réalisées en plusieurs points du milieu.

Selon un premier mode de réalisation de l'invention, la distribution spatiale de phase $\alpha_n$ ($1 \leq n \leq N$) est modifiée par itérations successives des phases appliquées sur au moins une pluralité de sources, la distribution de phase optimale $\alpha_n$ étant choisie comme l'itération qui génère la perturbation maximale.

Selon cette caractéristique, une itération empirique est réalisée et le choix de la distribution de phase optimale à appliquer à la pluralité de sources testée est fait parmi les distributions de phase testées lors de l'itération. La mesure de l'intensité de la perturbation induite dans la zone focale à chaque itération et le choix de la distribution de phase sur la pluralité de sources résultant en la plus importante perturbation permettent de choisir la distribution de phase ou d'amplitude optimale pour la pluralité de sources.

Selon une caractéristique avantageuse, , la distribution de phase $\alpha_n$ étant décomposée en une base de K vecteurs $V_k$ ($1 \leq k \leq K$) avec $\alpha_n = \sum_{k=1}^{K} d_k V_{kn}$ où $d_k$ est un nombre réel, chaque vecteur particulier $V_k$ décrivant les phases émises par les N sources, les modifications de phase $\alpha_n$ ($1 \leq n \leq N$) sont réalisées sur l'ensemble des N sources par $S_k$ modifications de la valeur de K $d_k$ pour chaque vecteur $V_k$, M étant alors égal à $\sum_{k=0}^{K} S_k$.

Une telle caractéristique permet de sélectionner et/ou de pondérer simultanément toutes les sources pour constituer la pluralité de sources. Cette approche possède deux avantages. D'une part, la définition de vecteurs à partir des variations spatiales des aberrations permet d'effectuer, selon le procédé de l'invention, des modifications de phase tenant directement compte des spécificités de ces variations. D'autre part, en émettant avec l'ensemble des émetteurs, on évite de faire des modifications de phase qui n'auraient pas d'impact perceptible sur l'amplitude de l'intensité de la perturbation mesurée. Cette approche permet donc de réduire considérablement le nombre d'itérations modificatives du procédé. La réalisation d'une itération du nombre $d_k$ pour chaque vecteur $V_k$ permet de déterminer un nombre $d_k$ générant une distribution de phase optimale pour le vecteur $V_k$. La réédition de l'itération pour $d_{k+1}$ associée au vecteur $V_{k+1}$ suivant permet de converger vers une focalisation optimale sur l'ensemble des sources du réseau.

Avantageusement, les modifications de la distribution de phase $\alpha_n$ sont réalisées dans un sous-espace de K' (K'<K) vecteurs de la base $V_k$ avec $\alpha_n = \sum_{k=1}^{K'} d_k V_{kn}$, M étant alors égal à $\sum_{k=0}^{K'} S_k$.

Une telle caractéristique permet de sélectionner un certain nombre de fréquences spatiales parmi un ensemble de vecteurs décrivant un espace de fréquences spatiales réduit. Cela permet de réduire la durée de l'optimisation de la distribution de phase.

Dans une implémentation avantageuse, les vecteurs $V_k$ sont définis à partir de K fonctions périodiques de l'espace, chaque coefficient $V_{kn}$ étant déterminé par la valeur d'une fonction k ($1 \leq k \leq K$) relative à la position de la source n.

Les fonctions périodiques qui peuvent être des fonctions sinusoïdales permettent de décrire simplement un espace particulier de fréquences spatiales.

Selon une caractéristique additionnelle avantageuse de réalisation, la distribution spatiale d'amplitude $A_n$ ($1 \leq n \leq N$) est modifiée par modifications successives sur au moins une pluralité de sources, la distribution d'amplitude

optimale $A_{n_{opt}}$ étant choisie comme l'itération qui génère la perturbation maximale.

Cette caractéristique additionnelle permet d'ajouter une optimisation de l'amplitude en fonction des fréquences spatiales en plus de l'optimisation des phases.

Les modifications de la distribution d'amplitude selon la caractéristique additionnelle peuvent être réalisées successivement, alternativement ou simultanément avec la modification de la distribution de phase.

Selon un second mode de réalisation, les modifications des amplitudes $A_n$ et des phases $\alpha_n$ sont une combinaison de P vecteurs $H_p$ définis sous une forme matricielle $H_{pn}$ (1≤p≤P et 1≤n≤N) avec $A_n.e^{j\alpha_n} = \sum_{p=1}^{P} c_p H_{pn}$, où chaque valeur $c_p$ est un nombre complexe.

Avec une telle caractéristique, chaque modification de la distribution de phase et/ou d'amplitude correspond à un changement de la valeur du nombre complexe $c_p$. Comme cette modification est alors commune à plusieurs sources désignées par l'intermédiaire du vecteur $H_p$, cette caractéristique permet de générer une perturbation détectable.

Avec cette caractéristique, la distribution de phase optimale est déterminée à l'aide des mesures de perturbation utilisées en tant que déterminants dans les équations théoriques liant la phase et l'énergie de la perturbation.

La matrice peut être une matrice orthogonale, par exemple une matrice d'Hadamard.

Selon une caractéristique avantageuse, les M modifications de la distribution de phase et/ou d'amplitude sont réalisées par $R_p \geq 2$ modifications de la valeur de phase $x_r$ lors de l'émission de la somme $H_{p_1 n} + H_{pn}.e^{jx_r}$ sur chaque source n correspondant à la somme d'au moins deux vecteurs $H_{p1}$ et $H_p$ (1≤p≤P) de la base dont l'un est déphasé d'une phase $x_r$, M étant alors égal à $\sum_{p=1}^{P} R_p$, la distribution de phases et/ou d'amplitudes optimale étant déterminée grâce à la mesure des perturbations $I_m$ obtenues pour chaque somme de vecteurs émise et par inversion de la matrice $H_{pn}$.

Cette caractéristique, consistant à appliquer un déphasage $x_r$ à un des deux vecteurs, permet, avec l'aide des équations théoriques, d'obtenir la valeur du décalage de phase et d'amplitude $B_p.e^{j\beta_p}$ introduit par l'élément aberrateur entre les deux vecteurs d'émission différents $H_{p1}$ et $H_p$, ceci en réalisant plusieurs mesures des perturbations engendrées par l'émission successive de la somme $H_{p_1} + H_p.e^{jx}$ pour plusieurs valeurs de x.

La déduction de la distribution des décalages de phase $\varphi_n$ est obtenue par inversion de la matrice $H_{p_n}$ et en utilisant les $B_p.e^{j\beta_p}$ obtenus pour chaque vecteur $H_p$ :

$$A_n.e^{j\varphi_n} = inv(H_{pn})B_p e^{j\beta_p}$$

La distribution de phase optimale $\alpha_{n_{opt}}$ à émettre sur chacune des sources est ensuite obtenue directement par inversion de la phase : $e^{j\alpha_n} = e^{-j\varphi_n}$

Dans une application avantageuse de l'invention, le milieu de focalisation est un milieu biologique.

Selon l'invention, la perturbation $I_m$ induite et mesurée dans le milieu peut être choisie parmi des déplacements locaux, des vélocités locales, des contraintes, des variations de température locales dans le milieu.

En fonction de la nature des ondes et de celle du milieu, un de ces types de perturbations sera privilégiée. Par exemple, dans le cas d'ondes ultrasonores, le procédé peut être mis en oeuvre avec des ondes d'une intensité telle qu'elle permet de générer une poussée dans le milieu due à la force de radiation ultrasonore mais d'une intensité telle qu'elle ne génère pas de dommages dans le milieu, comme c'est le cas, en revanche, lors de la thérapie.

Selon une autre caractéristique particulière de l'invention, la mesure de l'intensité de la perturbation induite $I_m$ est réalisée à l'aide d'une modalité d'imagerie.

L'utilisation d'une modalité d'imagerie pour suivre l'intensité de la perturbation permet de suivre directement et de manière fiable la localisation du point focal par rapport à des structures imagées du milieu. Dans le cas de la thérapie, cela permet aussi de voir les lésions en même temps et de suivre ensuite, le cas échéant, l'effet thérapeutique en temps réel d'un traitement. Cela évite en outre d'avoir à déplacer le patient entre une phase d'imagerie et une phase de traitement. Cette caractéristique permet bien sûr aussi la localisation directe de la zone à traiter sans avoir à recaler le patient dans un référentiel correspondant à une étape d'imagerie préalable.

Par exemple, les déplacements micrométriques induits par le faisceau ultrasonore seront suivis par imagerie à résonance magnétique. De cette manière, il est possible de rendre plus souples et plus rapides les protocoles de traitement des tumeurs cérébrales en permettant le guidage et le suivi du traitement grâce à une unique modalité d'imagerie.

La modalité d'imagerie peut être choisie parmi les modalités d'imagerie à résonance magnétique, les modalités d'imagerie ultrasonore, les modalités d'imagerie tomographique par rayons X, les modalités d'imagerie optique.

La modalité d'imagerie à résonance magnétique est particulièrement adaptée pour suivre les évolutions de tissus organiques et est préférentiellement choisie dans les applications médicales où, outre le fait que cette imagerie permet de mettre en oeuvre l'invention, elle permet successivement et/ou simultanément de suivre l'effet d'un traitement ou encore elle permet l'imagerie, simultanée et d'autant plus précise que la focalisation d'ondes est meilleure, de la zone d'intérêt que l'on souhaite examiner.

L'invention est ainsi mise en oeuvre, de manière tout à fait originale, pour focaliser des ondes ultrasonores dans un milieu qui est parallèlement imagé par imagerie à résonance magnétique. Ces deux modalités présente l'avantage majeur d'être tout à fait compatibles et de pouvoir être réalisées simultanément.

Avantageusement, la mesure de la perturbation $I_m$ induite dans la zone d'intérêt est réalisée par imagerie de la zone d'intérêt elle-même.

Dans une mise en oeuvre préférée de l'invention, la perturbation $I_m$ induite et mesurée dans le milieu est due à une force de radiation ultrasonore, sonore ou électromagnétique.

Une telle perturbation peut être mesurée en tant que déplacement mais également en tant qu'échauffement. Le type de mesure pourra notamment être choisi en fonction de la nature du milieu.

L'invention vise en outre un système émetteur comprenant

a) un réseau de N sources aptes à émettre des ondes focalisées $A_n.e^{j\alpha_n}$ présentant une distribution spatiale de phase $\alpha_n$ et d'amplitude $A_n$ ($1 \le n \le N$) vers une zone d'intérêt d'un milieu, le système mettant en oeuvre un procédé d'optimisation de la focalisation dans le milieu selon l'invention utile lorsque les ondes focalisées sont émises au travers d'un élément introducteur d'aberrations introduisant un décalage de phase initialement indéterminé, le système comprenant pour cela des moyens pour, lors de l'émission d'ondes focalisées dans le milieu,

b) modifier M fois la distribution spatiale de phase $\alpha_n$ et/ou l'amplitude $A_n$ ($1 \le n \le N$) des ondes émises simultanément par une pluralité de sources du réseau, chaque modification correspondant à des modifications simultanées des phases et/ou amplitudes sur cette pluralité de sources du réseau,

c) mesurer une perturbation $I_m$ ($1 \le m \le M$) induite par les ondes dans la zone d'intérêt à chaque modification m de la distribution des phases $\alpha_n$ et/ou d'amplitudes $A_n$,

d) déduire, à partir des mesures de perturbation $I_m$, une distribution optimale de phase d'émission $\alpha_{n_{opt}}$ et/ou une distribution d'amplitude $A_{n_{opt}}$ maximisant la perturbation induite dans la zone d'intérêt.

Selon une caractéristique avantageuse, les sources sont des émetteurs ultrasonores.

Selon une implémentation préférée, les différentes étapes du procédé sont déterminées par des instructions de programmes d'ordinateurs.

En conséquence, l'invention vise aussi un programme d'ordinateur sur un support d'informations, ce programme étant susceptible d'être mis en oeuvre dans un système émetteur d'ondes focalisées ce programme comportant des instructions adaptées à la mise en oeuvre des étapes du procédé selon l'invention.

Ce programme peut utiliser n'importe quel langage de programmation, et être sous la forme de code source, code objet, ou de code intermédiaire entre code source et code objet, tel que dans une forme partiellement compilée, ou dans n'importe quelle autre forme souhaitable.

L'invention vise aussi un support d'informations lisible par un système selon l'invention, et comportant des instructions d'un programme d'ordinateur tel que mentionné ci-dessus.

Le support d'informations peut être n'importe quelle entité ou dispositif capable de stocker le programme. Par exemple, le support peut comporter un moyen de stockage, tel qu'une ROM, par example un CD ROM ou une ROM de circuit microélectronique, ou encore un moyen d'enregistrement magnétique, par exemple une disquette (floppy disc) ou un disque dur ou encore une carte mémoire.

D'autre part, le support d'informations peut être un support transmissible tel qu'un signal électrique ou optique, qui peut être acheminé via un câble électrique ou optique, par radio ou par d'autres moyens. Le programme selon l'invention peut être en particulier téléchargé sur un réseau de type Internet.

Alternativement, le support d'informations peut être un circuit intégré dans lequel le programme est incorporé, le circuit étant adapté pour exécuter ou pour être utilisé dans l'exécution du procédé en question.

Brève description des dessins

D'autres caractéristiques et avantages de la présente invention ressortiront de la description faite ci-dessous, en référence aux dessins annexés qui en illustrent un exemple de réalisation dépourvu de tout caractère limitatif. Sur les figures :

- la figure 1 décrit une sonde ultrasonore telle qu'avantageusement utilisée pour mettre en oeuvre l'invention,
- la figure 2 présente une situation d'optimisation de la focalisation selon le procédé de l'invention,
- la figure 3 représente le domaine de fréquence des variations spatiales significatives des aberrations engendrées par la traversée de l'élément introducteur d'aberrations,
- la figure 4 donne un exemple de correction des aberrations engendrées par la traversée de l'élément introducteur d'aberrations par un procédé d'optimisation de focalisation selon l'invention.

Description détaillée d'un mode de réalisation

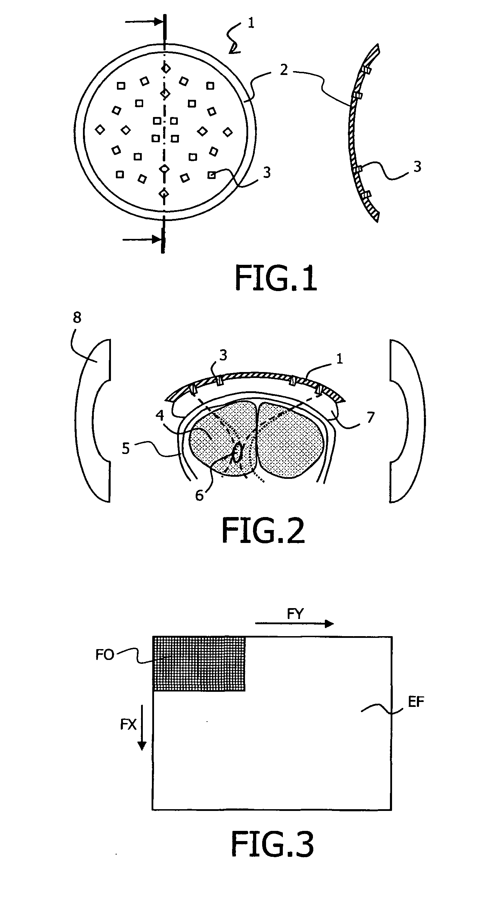

La figure 1 représente une sonde ultrasonore 1 telle qu'avantageusement utilisée pour mettre en oeuvre l'invention. La sonde ultrasonore 1 présentée sur cette figure est particulièrement destinée à permettre une optimisation de la focalisation d'ondes ultrasonores dans le cerveau.

En effet, l'invention est particulièrement intéressante dans ce contexte puisque l'os du crâne introduit inévitablement des aberrations de phase au sein d'un faisceau focalisé. Ces aberrations empêchent une bonne localisation de la zone focale ce qui peut s'avérer particulièrement gênant dans les applications médicales où une grande précision est requise.

L'os du crâne demeure cependant relativement transparent pour les ultrasons. Il y a donc lieu de développer des solutions pour corriger les aberrations induites par la traversée de l'os du crâne afin de pouvoir utiliser les ondes ultrasonores, malgré les aberrations, pour imager ou soigner une zone du cerveau.

La sonde ultrasonore 1 comprend ainsi un châssis 2 formant une portion de sphère de rayon de courbure choisi en fonction de l'application visée. Dans l'exemple choisi, visant l'imagerie et la thérapie du cerveau, le rayon de courbure sera par exemple égal à 120 mm. Le châssis 2 porte un nombre donné N de transducteurs piézoélectriques 3.

Dans une réalisation pratique, N=512 transducteurs piézoélectriques 3 sont ainsi installés sur le châssis 2. Ils constituent autant de sources d'ondes. Pour la simplicité de la représentation, seuls quelques transducteurs sont représentés sur la figure 1 qui n'est qu'illustrative. Chaque élément transducteur 3 individuel est apte à émettre, de manière continue et/ou discontinue, une onde ultrasonore. Dans un exemple de réalisation, chaque élément individuel 3 possède un diamètre de 8 mm et émet de manière continue une sinusoïde à la fréquence centrale de 1 MHz.

Classiquement, lorsqu'une sonde émet un faisceau d'ondes ultrasonores, les phases des ondes émises par chaque élément transducteur de la sonde sont calculées individuellement afin de réaliser une focalisation dans une zone dite focale du milieu.

Les phases initialement calculées pour réaliser cette focalisation ne peuvent cependant pas tenir compte des aberrations introduites par les hétérogénéités du milieu dans lequel les ondes vont focaliser. Ces aberrations sont en effet inconnues. L'invention permet d'atténuer voire de supprimer l'effet de ces aberrations.

La figure 2 représente l'utilisation d'une sonde ultrasonore 1, telle que présentée sur la figure 1, pour l'imagerie ou la thérapie au sein d'un milieu 4, en l'occurrence le cerveau, enveloppé dans sa boîte crânienne, élément introducteur d'aberrations 6. La transmission des ondes des transducteurs 3 vers la boite crânienne 5 et le cerveau 4 est assurée par un milieu 7 d'impédance adaptée.

La focalisation souhaitée, illustrée par des lignes de tirets, définit une zone focale 6. En l'absence de correction de phase, on observe une dégradation de la focalisation, représentée par des lignes de points sur la figure 2. Cette dégradation est due à la présence de l'élément introducteur d'aberrations 5 constitué par la boite crânienne placé sur le trajet du faisceau ultrasonore émis par la sonde 1.

Au niveau de la zone focale 6, la contribution p du n-ième élément transducteur 3 au champ acoustique total obtenu dans la zone focale 6, après propagation à travers l'os crânien, s'écrit :

$$p_n(t) = A_n D_n e^{j\alpha_n} e^{-jK_w R_c} e^{j2\pi f t} e^{j\varphi_n} \, ,$$

où

$\varphi_n$ est le décalage de phase introduit par le milieu introducteur d'aberrations 5, $A_n$ est l'amplitude à l'émission, $\alpha_n$ représente la phase correspondant, dans la distribution de phase, à l'élément transducteur n, $D_n$ est un facteur d'atténuation introduit par les phénomènes d'absorption lors de la traversée de la boîte crânienne 5 et par la perte d'amplitude par diffraction, f est la fréquence de l'onde émise, $K_w$ est le vecteur d'onde et $R_c$ est le rayon de courbure du système d'émission.

Le champ acoustique total est donc donné par l'équation :

$$P(t) = e^{j2\pi ft} e^{-jK_w \cdot R_c} \sum_n A_n D_n e^{j\varphi_n} e^{j\alpha_n}$$

Ce champ acoustique total génère une perturbation *I* dans le milieu 4.

Dans l'invention, l'intensité *I* de la perturbation est suivie à l'aide de mesures effectuées dans le milieu 4, au niveau de la zone focale 6. La mesure de l'intensité de la perturbation *I* est avantageusement réalisée grâce à une modalité d'imagerie 8, par exemple l'imagerie par résonance magnétique ou encore l'échographie.

Néanmoins, ces modalités d'imagerie ne permettent pas de mesurer directement et à distance la surpression induite par le champ ultrasonore. En revanche, dans le cas d'ondes ultrasonores, l'énergie ultrasonore locale peut être directement estimée.

Cette énergie ultrasonore est proportionnelle au carré de la surpression induite par le champ ultrasonore. On note cependant que l'accès à l'énergie ultrasonore locale ne permet pas d'accéder directement à l'information de phase et donc au décalage de phase introduit. Aussi, avec ces modalités d'imagerie, seule une optimisation par mesure d'amplitude est donc possible.

Dans un exemple de réalisation avantageux, l'énergie ultrasonore locale est suffisante pour provoquer un déplacement au sein du tissu mais sans y engendrer de dégradations. Ce déplacement, résultant de l'application du champ ultrasonore dans la zone focale, est alors mesuré grâce à l'imagerie des tissus situés dans la zone focale.

Une telle mesure est connue de l'homme du métier qui peut se reporter au document Viscoelastic shear properties of in vivo breast lesions measured by MR elastography, Sinkus R, Tanter M, Xydeas T, et al., MAGNETIC RESONANCE IMAGING 23 (2): 159-165 Sp. Iss. SI FEB 2005 pour en trouver les caractéristiques dans le cas de l'imagerie par résonance magnétique.

En particulier, lorsqu'une modalité d'imagerie ultrasonore est utilisée pour mesurer la perturbation, toutes les techniques connues de mesure de déplacement dont les mesures Doppler, les techniques d'inter-corrélation de signaux, les mesures de variations de phase dans le domaine de Fourier etc. pourront être mises en oeuvre.

Lorsqu'une modalité d'imagerie par résonance magnétique est utilisée pour suivre la perturbation *I*, le déplacement au foyer peut être suivi en utilisant des séquences d'imagerie à résonance magnétique sensibles au mouvement en fonction du temps. Ces séquences d'imagerie à résonance magnétique permettent d'accéder à des mouvements échantillonnés sur un temps très court de 1 à 10 millisecondes.

Par exemple, des séquences similaires aux séquences bien connues en IRM de diffusion pourront être mises en oeuvre. Il est en particulier connu qu'en utilisant une intégration dans le temps des déplacements mesurés pendant le temps d'application du gradient de champ magnétique lors d'une séquence IRM de diffusion modifiée, une cartographie bidimensionnelle du déplacement généré par un transducteur ultrasonore peut être obtenue. Dans chaque voxel de cette cartographie, le déplacement correspond à une intégration sur un temps court des déplacements de ce voxel pendant la poussée.

On déduit ensuite, à partir des déplacements observés, la force de radiation ultrasonore induite dans la zone focale. Sa densité volumique de force est alors exprimée par la formule :

$$\mathbf{f} = 2aI\mathbf{e}_z / c ,$$

où

*I* est l'intensité acoustique, a est le coefficient d'absorption ultrasonore du milieu, c est la vitesse de propagation du son dans les tissus, et $e_z$ est le vecteur de direction de propagation.

En supposant que l'onde est localement plane au foyer, l'intensité acoustique dans la zone focale est alors

donnée par $I = \dfrac{\langle P^2 \rangle}{Z} = \dfrac{1}{2Z}\left|\sum_{n=1}^{N} A_n D_n e^{i\varphi_n} e^{i\alpha_n}\right|^2$, où <P²> est la moyenne temporelle de l'énergie acoustique exprimée ci-dessus et Z est l'impédance acoustique du milieu.

Dans les exemples de réalisation décrits dans la suite, l'énergie acoustique *I* est choisie comme étant la mesure représentative de la perturbation.

L'invention utilise les fluctuations des amplitudes de la perturbation $I_m$ mesurée, par exemple l'intensité acoustique, dans la zone focale 6 lorsque les retards de phase sur chacun des éléments transducteurs 3 de la sonde ultrasonore 1 sont modifiés m fois selon le procédé de l'invention.

Les ondes focalisées peuvent aussi être choisies de manière à ce qu'elles provoquent une élévation de température au sein du milieu. Dans ce cas, la mesure de la perturbation par élévation de température peut notamment être réalisée par imagerie à résonance magnétique en utilisant d'autres séquences spécifiques.

Pratiquement, l'invention propose de modifier les décalages de phase sur un ensemble de transducteurs tout en mesurant les perturbations $I_m$ induites dans la zone d'intérêt afin de déterminer une distribution de phase optimale du point de vue de la focalisation.

Avantageusement, une ou des mesure(s) additionnelle(s) de perturbation(s) $I_{m_e}$ induite(s) à l'extérieur de la zone d'intérêt 6 sont également réalisées. Cela est en particulier aisé lorsqu'une modalité d'imagerie à résonance magnétique est utilisée puisque cette imagerie permet d'observer des phénomènes sur une zone relativement étendue

Deux modes de réalisation sont présentés dans la suite. Tous deux utilisent le principe d'une modification intentionnelle et connue de la distribution de phase afin de déterminer la distribution de phase optimale à l'aide de la mesure de la perturbation engendrée dans le milieu.

Le premier mode de réalisation utilise une modification itérative des phases au sein de la distribution de phase. La distribution de phase est alors avantageusement décrite à l'aide de vecteurs spatiaux décrivant les phases des ondes émises pour chaque source n (1≤n≤N) du réseau de N sources. La phase K de l'élément n s'exprime alors par exemple :

$\alpha_n = \sum_{k=1}^{K} d_k V_{kn}$ où $d_k$ est un nombre réel.

Cette base est préférentiellement choisie pour correspondre aux fréquences spatiales les plus couramment observées au sein des variations des aberrations introduites par l'élément introducteur d'aberrations.

Or il se trouve que, pour les applications médicales visées, les aberrations de phase introduites par les structures biologiques, varient relativement lentement dans l'espace. C'est en particulier le cas pour le crane humain. La solution optimale des décalages de phase peut donc être recherchée en modifiant des valeurs associées à un nombre limité de vecteurs correspondant à un nombre limité de fréquences spatiales. Le principe permet donc de modifier la phase sur plusieurs transducteurs, voire la totalité, en ne modifiant que la valeur associée à un des vecteurs.

Une décomposition des variations spatiales des aberrations typiquement introduites par la boite crânienne est par exemple réalisée en faisant une transformation en cosinus discret (en anglais : Discrete Cosine Transform ou DCT) sur la base des fréquences spatiales FX et FY. Une telle décomposition est illustrée sur la figure 3. On constate, sur cette figure, que l'espace des fréquences occupées FO sur l'espace des fréquences EF par les fréquences spatiales les plus communes se situe dans les basses fréquences.

Ainsi, une nouvelle base de vecteurs, différente de la base canonique désignant chaque transducteur un par un, est alors avantageusement définie en tenant compte des spécificités fréquentielles des aberrations introduites : chaque couple de fréquences spatiales non nulles (FX,FY) respectivement suivant les axes X et Y du réseau de transducteurs 3 est choisi et le vecteur spatial correspondant est construit.

Dans un exemple de réalisation, le vecteur est constitué de la manière suivante :

$$V_k = \begin{pmatrix} \cos(P_{k_x}\pi x_1).\cos(P_{k_y}\pi y_1) \\ \cos(P_{k_x}\pi x_2).\cos(P_{k_y}\pi y_2) \\ \cos(P_{k_x}\pi x_3).\cos(P_{k_y}\pi y_3) \\ \ldots \\ \cos(P_{k_x}\pi x_N).\cos(P_{k_y}\pi y_N) \end{pmatrix}$$

avec $P_{k_x}$ et $P_{k_y}$ entiers permettant de

décrire un ensemble de fréquences spatiales, $(x_n, y_n)$ étant la position du transducteur n sur la sonde repérée dans un repère bidimensionnel. $P_{k_x}$ et $P_{k_y}$ décrivent par exemple l'ensemble des entiers entre 2 et 10. Ils peuvent être égaux permettant de décrire les mêmes fréquences spatiales FX=FY sur les deux dimensions ou différents décrivant des fréquences FX et FY distinctes.

La distribution de phase est ensuite modifiée M fois sur la base de ces vecteurs sinusoïdaux, ce nombre pouvant être inférieur aux nombres N de transducteurs.

Comme la phase de la source n s'écrit alors $\alpha_n = \sum_{k=1}^{K} d_k V_{kn}$ où $d_k$ est un nombre réel, optimiser la distribution de phase consiste donc à déterminer un ensemble de valeurs $d_k$ chacune associée à un vecteur $V_k$. Pour cela, on applique successivement plusieurs valeurs de $d_k$ à chacun de ces vecteurs, par exemple en incrémentant S fois la valeur $d_k$ par exemple par incréments de 0.1 radian. A chaque modification, une mesure de la perturbation engendrée est réalisée.

Le nombre S peut être identique pour tous les vecteurs $V_k$ ou dépendre du vecteur $V_k$ considéré. Il peut alors être noté $S_k$.

Une fois la valeur de $d_k$ résultant en la plus grande perturbation déterminée pour un vecteur, on réalise la même opération pour un autre vecteur en appliquant la valeur optimale au vecteur précédent en tant que loi de correction et ainsi de suite.

Cette opération est réalisée sur tout ou partie des vecteurs $V_k$ construits à partir de couples de fréquences spatiales (FX,FY). On réalise ainsi $M = \sum_{k=1}^{K} S_k$ modifications. Si le nombre d'incrémentation S est identique pour tous les vecteurs $V_k$, alors le nombre total de modifications est égal à M=S.K.

Pour chaque modification m, au moins une mesure d'une perturbation $I_m$ est réalisée. La valeur $d_k$ correspondant à la modification m, pour laquelle la perturbation $I_m$ maximale est observée, est sélectionnée.

Dans ce mode de réalisation, en optimisant ainsi la distribution de phase pour chacun de ces vecteurs $V_k$ décrivant chacun un couple de fréquences particulières (FX,FY) de l'espace des fréquences spatiales, on optimise la focalisation successivement sur l'ensemble des fréquences spatiales des aberrations.

En utilisant ensuite chaque distribution optimale, sur chaque vecteur, on obtient une distribution spatiale optimale $\alpha_{n_{opt}}$ sur l'ensemble des fréquences spatiales concernées.

En effet, à l'issue de l'optimisation sur chaque vecteur $V_k$, la correction de distribution de phase optimale est alors la somme des vecteurs auxquels sont associées les valeurs $d_k$ engendrant une perturbation maximale. En combinant l'effet optimisé de l'ensemble des vecteurs, la perturbation maximale est alors obtenue lorsque chaque transducteur présente la phase suivante : $\alpha_{n_{opt}} = \sum_{k=1}^{K} d_k V_{kn}$ avec $d_k$, valeur optimisée. Une partie des aberrations introduites par l'élément aberrateur sont alors corrigées. Au besoin, il peut être envisagé de recommencer l'ensemble des itérations pour affiner l'optimisation.

Par ailleurs, lorsqu'une ou des mesure(s) de perturbation $I_{me}$ sont effectuées à l'extérieur de la zone d'intérêt 6, ces mesures peuvent être utilisées pour choisir une distribution de phase optimale permettant, outre la maximisation de la perturbation $I_m$ dans la zone d'intérêt 6, la minimisation de la perturbation $I_{me}$ hors de la zone d'intérêt 6. Une telle minimisation peut, en particulier, être l'objet de la seconde itération permettant d'affiner l'optimisation.

En outre, dans la mesure où la boîte crânienne 5 introduit aussi des aberrations d'amplitude, outre l'optimisation de la distribution de phase présentée ci-dessus, une correction du second ordre utilisant une modification de l'amplitude peut également être appliquée.

Cette correction est avantageusement réalisée après une première optimisation de la distribution de phase. Dans ce cas, une seconde optimisation de la distribution de phase peut avantageusement être réalisée à la suite de l'optimisation de l'amplitude.

Un exemple de réalisation de cette correction du second ordre consiste à faire varier, par paliers successifs, généralement entre 0,1 et 1, un coefficient d'amplitude $A'_k$ appliqué à chaque onde construite à partir de la distribution de N phase décrite par chaque vecteur $V_k$ de la manière suivante : $A'_k \cdot \sum_{n=1}^{N} A_n e^{j\alpha_n}$ où $A_n$ est l'amplitude par défaut de chaque source.

De façon analogue à la variation de phase, plusieurs émissions avec S' différents coefficients $A'_k$ sont effectuées

pour maximiser soit la quantité I, soit la quantité suivante $\dfrac{I}{\sum\limits_{k=1}^{K} ACk^2}$, ceci afin d'optimiser le rapport entre l'intensité au foyer et l'intensité émise par les sources. Pour illustration, dès lors que S' incrémentations sont réalisées pour tous les vecteurs, on réalise alors un nombre de modifications supplémentaire égale à M=S'.K.

Aussi, en testant successivement plusieurs distributions de phase et plusieurs facteurs d'amplitude, pour chacun des vecteurs décrivant l'espace des fréquences spatiales des aberrations, il est possible d'accéder à un optimum de perturbation au niveau de la zone focale et, le cas échéant, à un minimum de perturbation hors de la zone focale, révélant une optimisation de la focalisation malgré les aberrations de phase induites par la boîte crânienne 5.

L'utilisation de vecteurs décrivant l'espace des fréquences spatiales des aberrations attendues par l'élément aberrateur permet de modifier la phase sur l'ensemble des transducteurs en même temps et, donc, de fournir au milieu une énergie suffisante pour que le différentiel de perturbation soit mesurable.

En effet, les déplacements des tissus induits par la force de radiation ultrasonore sont généralement très faibles, de l'ordre de 10 à 100 $\mu$m. Ainsi, une limitation majeure correspond au déplacement minimal qui peut être mesuré par la modalité d'imagerie. Ce déplacement minimal est typiquement de l'ordre de 1 $\mu$m. L'énergie fournie au milieu et la perturbation engendrée doit donc être suffisante pour être décelable au vu de la sensibilité de la mesure.

En utilisant des modifications de phase sur l'ensemble des sources, l'invention permet d'envoyer suffisamment d'énergie dans le milieu non seulement pour générer une perturbation décelable mais aussi pour que les différences d'énergie associées aux différentes combinaisons de décalages de phase soient décelables pour permettre l'optimisation.

La modalité d'imagerie choisie et le dispositif la mettant en oeuvre déterminent la durée nécessaire pour faire une mesure correspondant à une itération de la distribution de phase. Par exemple, avec une technologie ultrasonore, cette durée est de l'ordre de 1 ms et avec, la technologie par imagerie à résonance magnétique, si l'on se restreint à la mesure en un voxel 3D unique, cette durée est de l'ordre de 10 ms. Ainsi, avec la technologie IRM, sur une durée totale maximale de 10 minutes, le nombre total d'itérations sera limité à 60000.

Le premier mode de réalisation décrit ci-dessus présente l'avantage d'utiliser un nombre réduit de fréquences spatiales, de l'ordre de 10 suivant chaque direction X et Y et de permettre ainsi une convergence itérative rapide vers une solution très proche de la correction optimale. Environ 1000 à 2000 itérations sont alors suffisantes. Avec les modalités d'imagerie classiques, cela correspond à un temps d'optimisation de l'ordre de 3 minutes tout à fait acceptable cliniquement.

Le fait de modifier la phase non pas transducteur par transducteur, mais sur la totalité des éléments ou, du moins sur une pluralité d'entre eux, à chaque étape de l'itération permet d'engendrer des modifications décelables de l'intensité acoustique au foyer et ainsi d'être moins tributaire de la sensibilité de la mesure de l'énergie ultrasonore dans la zone focale.

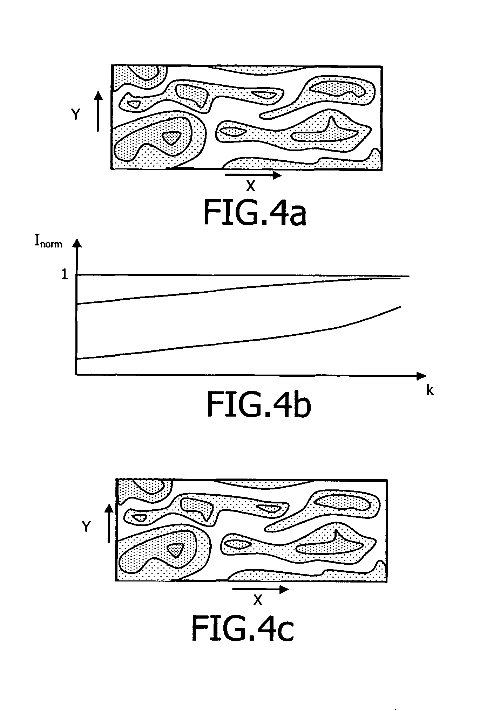

Dans la suite, est proposée une simulation du procédé réalisée avec un introducteur d'aberrations théorique représenté par une distribution spatiale d'aberrations de phase sur la figure 4a. Le processus converge en environ 4000 pas où sont testées 10 fréquences spatiales dans chaque direction (donc K=100=10*10) et S=30 décalages de phase pour chaque vecteur propre $V_k$. Sur la figure 4b, l'amplitude $I_{norm}$ de l'énergie acoustique normalisée par rapport à l'énergie optimale qui serait obtenue avec une correction parfaite est reportée en fonction du nombre k désignant chaque vecteur.

L'optimisation est ici réalisée deux fois successivement pour l'ensemble des vecteurs spatiaux. On réalise donc ici M=S.K modifications. On constate que l'amplitude $I_{norm}$ de l'énergie acoustique au foyer augmente de manière importante au cours du processus. Le procédé selon l'invention permet bien une convergence vers la focalisation parfaite.

La loi de correction finalement obtenue est présentée sur la figure 4c. Elle est très proche de la loi d'aberration théorique introduite pour la simulation et présentée sur la figure 4a.

Selon le premier mode de réalisation, l'invention est un procédé expérimental selon lequel on optimise une énergie acoustique générée dans un milieu afin de trouver une distribution de phase permettant de réduire l'influence des aberrations dans la focalisation des ondes.

Dans un second mode de réalisation de l'invention, une inversion directe à partir des mesures de perturbations permet de déterminer une distribution de phase optimale.

Il est en effet théoriquement possible d'estimer le déphasage optimal entre deux transducteurs à partir d'une pluralité de mesures de l'intensité d'une perturbation générée par l'interférence des ondes émises par ces deux transducteurs. En effet, l'intensité générée par la superposition des deux faisceaux est modulée en amplitude par le cosinus de la phase relative entre les deux transducteurs.

Ainsi, lorsque deux faisceaux monochromatiques $s_1(t) = ae^{i(\omega t)}$ et $s_2(t) = be^{i(\omega t + \varphi)}$ interfèrent en un point, l'intensité générée est :

$$I = a^2 + b^2 + 2ab\cos\varphi = A + B\cos\varphi$$

Il est donc possible de déterminer la phase φ par modulation d'intensité. Si on ajoute un déphasage x supplémentaire au signal $s_2$, on obtient une intensité modulée par un cosinus en fonction de x :

$$I(x) = A + B\cos(\varphi + x)$$

A partir d'au moins trois mesures de I(x) en modifiant x à chaque mesure, il est donc théoriquement possible de calculer A, B et $\varphi$.

La phase de l'onde émise sur chaque transducteur est donc modifiée au moins deux fois par des incréments x distincts.

Une manière de calculer ce déphasage consiste à réaliser R (R>1) mesures de l'intensité pour R modifications de phase $x_r$ et d'inverser directement le système d'équations linéaires à trois inconnues A, Bcos(φ), et Bsin(φ) :

$$I(x_r) = A + B.\cos(\varphi)\cos(x_r) - B.\sin(\varphi)\sin(x_r)$$

En particulier, si que pour chaque mesure de $I(x_r)$ on réalise également une mesure de $I(x_r + \pi)$ alors :

$$\sum_r \cos(x_r) = \sum_r \sin(x_r) = 0$$

On peut alors mesurer deux sommes :

$$\begin{cases} sc = \sum_r I_r \cos(x_r) = \sum \left[ A\cos(x_r) + B\cos(\varphi)\cos^2(x_r) - B\sin(\varphi)\sin(x_r)\cos(x_r) \right] \\ ss = \sum_r I_r \sin(x_r) = \sum \left[ A\sin(x_r) + B\cos(\varphi)\cos(x_r)\sin(x_r) - B\sin(\varphi)\sin^2(x_r) \right] \end{cases}$$

$$\begin{cases} sc = B\cos(\varphi)\sum \cos^2(x_r) - B\sin(\varphi)\sum \sin(x_r)\cos(x_r) \\ ss = B\cos(\varphi)\sum \sin(x_r)\cos(x_r) - B\sin(\varphi)\sum \sin^2(x_r) \end{cases}$$

Ce système linéaire de deux équations comporte deux inconnues $B\cos(\varphi)$ et $B\sin(\varphi)$. En résolvant simplement ce système on obtient l'information sur la phase $\varphi$ qui est le décalage de phase introduit par l'élément introducteur d'aberrations entre les deux transducteurs.

La base des N transducteurs réels est la base canonique. L'objectif selon l'invention est d'estimer les déphasages et amplitudes des transducteurs dans cette base canonique :

$$T_n = A_n e^{j\alpha_n}$$

Cependant, du fait de l'amplitude faible de chacun des transducteurs, il est difficile de mesurer la modulation d'intensité générée par deux transducteurs. L'invention consiste à effectuer un changement de base permettant de faire fonctionner simultanément une pluralité de transducteurs. De nombreuses bases sont possibles, par exemple la base d'Hadamard :

$$H_{pn} = \begin{bmatrix} 1 & 1 & 1 & 1 & 1 & 1 & 1 & 1 \\ 1 & -1 & 1 & -1 & 1 & -1 & 1 & -1 \\ 1 & 1 & -1 & -1 & 1 & 1 & -1 & -1 \\ 1 & -1 & -1 & 1 & 1 & -1 & -1 & 1 \\ 1 & 1 & 1 & 1 & -1 & -1 & -1 & -1 \\ 1 & -1 & 1 & -1 & -1 & 1 & -1 & 1 \\ 1 & 1 & -1 & -1 & -1 & -1 & 1 & 1 \\ 1 & -1 & -1 & 1 & -1 & 1 & 1 & -1 \end{bmatrix}$$

Soit $H = H_{pn}$(avec $1 \le p \le P$ et $1 \le n \le N$) la matrice de changement de base, l'amplitude et la phase émise par chaque vecteur $H_p$ de cette base peut donc s'exprimer comme un vecteur complexe décrivant les amplitudes et phase émises par chaque vecteur $H_p$: $H_p = H_{pn}T_n = B_p e^{j\beta_p}$ où $B_p$ et $\beta_p$ sont les valeurs de l'amplitude et de la phase appliquée sur chaque groupe de transducteurs du vecteur désigné par le vecteur $H_p$.

Et donc :

$$H_p = B_p e^{j\beta_p} = H_{pn}A_n e^{j\alpha_n}$$

Si on prend un vecteur de base $H_{p1}$ comme référence (phase=0 et amplitude=1), alors pour chaque vecteur $H_p$ ($1{\le}p{\le}P$), $\beta_p$ est le décalage introduit par l'élément aberrateur sur le vecteur $H_p$ par rapport au vecteur $H_{p1}$.

Dans ce cas, $B_p e^{j\beta_p}$ peut alors être estimé pour chacun des vecteurs $H_p$ par la méthode de modulation d'intensité en émettant successivement pour R valeurs de $x_r$ le vecteur somme ($H_{p_1} + H_p e^{jx_r}$) sur le réseau de N sources et en mesurant les perturbations induites $I_m(x_r)$ pour au moins deux (donc R$\ge$2) et préférentiellement trois (R=3) valeurs distinctes de $x_r$. L'amplitude et la phase telles qu'effectivement observées dans le milieu sont alors obtenues par rapport au vecteur de référence $H_{p1}$ pour chaque vecteur $H_p$.

Cette estimation de $B_p e^{j\beta_p}$ est alors réalisée pour chacun des P vecteurs $H_p$ en réalisant, pour chacun, R modifications de $x_r$. Ici, on a considéré que R est semblable pour tous les vecteurs.

Néanmoins, ce nombre de modifications R peut être une fonction du vecteur $H_p$ et donc noté $R_p$. En tout,

$$M = \sum_{p=1}^{P} R_p$$ modifications de la distribution de phase et/ou d'amplitude sont donc effectuées. Quand $Rp = R$ est le même pour tous les vecteurs $H_p$, alors M=P.R.

Ensuite, pour obtenir les phases et amplitudes introduites par l'aberrateur individuellement sur chacune des N sources, il faut revenir dans la base canonique. Pour cela, il suffit d'inverser la matrice $H$. Dans le cas où la matrice n'est pas inversible, il suffit d'utiliser la matrice pseudo-inverse obtenue par décomposition en valeurs singulières. On obtient alors l'amplitude et la phase introduite par l'aberrateur sur chacun des transducteurs de la base canonique :

$$A_n e^{j\varphi_n} = inv(H_{pn})B_p e^{j\beta_p}$$

La phase optimale d'émission $\alpha_{nopt}$ pour chacune des N sources est alors obtenue en inversant le décalage

de phase : $e^{j\alpha_{n_{opt}}} = e^{-j\varphi_n}$

Il est, ici aussi, possible d'utiliser des mesures de perturbations $I_{me}$ effectuées à l'extérieur de la zone d'intérêt 6. Dans ce cas, on cherchera à minimiser une telle perturbation $I_{me}$.

Après inversion de la matrice en utilisant les perturbations $I_{me}$, on connait les aberrations introduites par l'élément aberrateur et on peut les utiliser de manière à appliquer une distribution de phase à la fois optimale pour l'intensité de la perturbation $I_m$ dans la zone d'intérêt 6 et la minimisation de la perturbation $I_{me}$ à l'extérieur de la zone d'intérêt 6.

Avantageusement, la base H est choisie de telle sorte que le bruit de mesure n'est que peu amplifié par l'inversion de la matrice $H_{pn}$. Cela revient par exemple à choisir une matrice H pour laquelle les vecteurs propres de plus grand poids sont des vecteurs propres correspondant à l'émission d'une intensité importante sur le réseau de sources.

On remarque enfin que diverses mises en oeuvre peuvent être réalisées selon les principes de l'invention. Notamment, diverses bases vectorielles décrivant les fréquences spatiales particulières observées pour les aberrations de l'élément introducteur d'aberrations peuvent être utilisées.

## Revendications

1. Procédé d'optimisation de la focalisation d'ondes dans une zone d'intérêt [6] d'un milieu [4], les ondes étant émises par un réseau de N sources [3] vers le milieu [4] au travers d'un élément introducteur d'aberrations [5] introduisant un décalage de phase initialement indéterminé $\varphi_n$ (1≤n≤N), le procédé utilisant M-1 modifications successives de l'onde émise entrainant chacune une perturbation, les M perturbations sont mesurées, ces mesures étant utilisées pour déduire des caractéristiques de focalisation optimale, comprenant les étapes suivantes :

   a) émission, par le réseau de N sources [3], d'ondes $A_n.e^{j\alpha_n}$ (avec j2 = - 1) présentant une distribution spatiale de phase $\alpha_n$ et d'amplitude $A_n$ (1≤n≤N) et se propageant vers la zone d'intérêt [6] dans le milieu [4],

   b) M-1 (M>1) modification(s) de la distribution spatiale de phase $\alpha_n$ (1≤n≤N) ou M-1 (M>1) modification(s) de la distribution spatiale d'amplitude $A_n$ (1≤n≤N) ou M-1 (M>1) modification(s) de la distribution spatiale de phase et d'amplitude (1≤n≤N) des ondes émises simultanément par une pluralité de sources [3] du réseau, chaque modification correspondant à des modifications simultanées des phases et/ou amplitudes sur cette pluralité de sources [3] du réseau et engendrant chacune une étape a) d'émission,

   c) mesure de l'intensité d'au moins une perturbation $I_m$ (1≤m≤M) induite par les ondes dans la zone d'intérêt [6] à chaque modification m (1≤m≤M) de la distribution des phases $\alpha_n$ ou à chaque modification m (1≤m≤M) de la distribution d'amplitudes $A_n$, ou à chaque modification m (1≤m≤M) de la distribution de phase et d'amplitude,

   d) déduction, à partir des mesures d'intensité de perturbation $I_m$, d'une distribution optimale de phase d'émission $\alpha_{n_{opt}}$ ou une distribution optimale d'amplitude $A_{n_{opt}}$ ou une distribution optimale de phase et d'amplitude maximisant l'intensité de la perturbation $I_m$ induite dans la zone d'intérêt [6].

2. Procédé selon la revendication 1, **caractérisé en ce qu'**il comprend une étape c') de mesure d'au moins une perturbation $I_{me}$ en un point situé hors de la zone d'intérêt [6] à chaque modification m (1≤m≤M) de la distribution des phases $\alpha_n$ et/ou d'amplitudes $A_n$, l'étape d) étant alors une étape de déduction, à partir des mesures d'intensité de perturbation $I_m$ et des mesures d'intensité de perturbation $I_m$, d'une distribution optimale de phase d'émission $\alpha_{n_{opt}}$ ou une distribution optimale d'amplitude $A_{n_{opt}}$ ou d'une distribution optimale de phase d'émission $\alpha_{n_{opt}}$ et d'amplitude $A_{n_{opt}}$ maximisant l'intensité de la perturbation $I_m$ induite dans la zone d'intérêt [6] et minimisant l'intensité de la ou les perturbations $I_{me}$ hors de la zone d'intérêt.

3. Procédé selon la revendication 1, **caractérisé en ce que** la distribution spatiale de phase $\alpha_n$ (1≤n≤N) est modifiée par itérations successives des phases appliquées sur au moins une pluralité de sources [3], la distribution de phase optimale $\alpha_{n_{opt}}$ étant choisie comme l'itération qui génère l'intensité de perturbation maximale.

4. Procédé selon la revendication 3, **caractérisé en ce que**, la distribution de phase $\alpha_n$ étant décomposée en une base de K vecteurs $V_k$ (1≤k≤K) avec $\alpha_n = \sum_{k=1}^{K} d_k V_{kn}$ où $d_k$ est un nombre réel, chaque vecteur particulier $V_k$ décrivant les phases émises par les N sources [3], les modifications de phase $\alpha_n$ (1≤n≤N) sont réalisées sur l'ensemble des N sources par $S_k$ modifications de la valeur de $d_k$ pour chaque vecteur $V_k$ , M étant alors égal à $\sum_{k=0}^{K} S_k$ .

5. Procédé selon la revendication 4, **caractérisé en ce que** les modifications de la distribution de phase $\alpha_n$ sont réalisées dans un sous-espace de K' (K'<K) vecteurs de la base $V_k$ avec $\alpha_n = \sum_{k=1}^{K'} d_k V_{kn}$ , M étant alors égal à

$$\sum_{k=0}^{K'} S_k \; .$$

6. Procédé selon l'une des revendications 4 et 5, **caractérisé en ce que** les vecteurs $V_k$ sont définis à partir de K fonctions périodiques de l'espace, chaque coefficient $V_{kn}$ étant déterminé par la valeur d'une fonction k (1≤k≤K) relative à la position de la source n.

7. Procédé selon l'une des revendications 3 à 6, **caractérisé en ce que** la distribution spatiale d'amplitude $A_n$ (1≤n≤N) est modifiée par modifications successives sur au moins une pluralité de sources [3], la distribution d'amplitude optimale $A_{n_{opt}}$ étant choisie comme l'itération qui génère la perturbation maximale.

8. Procédé selon la revendication 7, **caractérisé en ce que** les modifications de la distribution d'amplitude sont réalisées successivement, alternativement ou simultanément avec la modification de la distribution de phase.

9. Procédé selon la revendication 1, **caractérisé en ce que** les modifications des amplitudes $A_n$ et des phases $\alpha_n$ sont une combinaison de P vecteurs $H_p$ définis sous une forme matricielle $H_{pn}$ (1≤p≤P et 1≤n≤N) avec $A_n . e^{j\alpha_n} = \sum_{p=1}^{P} c_p H_{pn}$ , où chaque valeur $c_p$ est un nombre complexe.

10. Procédé selon la revendication 9, **caractérisé en ce que** la matrice $H_{pn}$ est orthogonale.

11. Procédé selon la revendication 10, **caractérisé en ce que** la matrice $H_{pn}$ est une matrice d'Hadamard.

12. Procédé selon l'une des revendications 9 à 11, **caractérisé en ce que** les M modifications de la distribution de phase ou de la distribution d'amplitude ou de la distribution de phase et d'amplitude sont réalisées par $R_p \geq 2$ modifications de la valeur de phase $x_r$ lors de l'émission de la somme $H_{p_1 n} + H_{pn} . e^{jx_r}$ sur chaque source n correspondant à la somme d'au moins deux vecteurs $H_{p1}$ et $H_p$ (1≤p≤P) de la base dont l'un est déphasé d'une phase $x_r$, M étant alors égal à $\sum_{p=1}^{P} R_p$ , la distribution de phases et d'amplitudes optimales étant déterminée grâce à la mesure d'intensité des perturbations $I_m$ obtenues pour chaque somme de vecteurs émise et par inversion de la matrice $H_{pn}$.

13. Procédé d'optimisation de focalisation selon l'une des revendications précédentes, **caractérisé en ce que** les ondes émises sont acoustiques, ultrasonores, électromagnétiques ou optiques.

14. Procédé d'optimisation de focalisation selon l'une des revendications précédentes, **caractérisé en ce que** le milieu de focalisation [4] est un milieu biologique.

15. Procédé d'optimisation de focalisation selon l'une des revendications précédentes, **caractérisé en ce que** la perturbation $I_m$ induite et mesurée dans le milieu est choisie parmi les déplacements locaux, les vélocités locales, les contraintes, les variations de température induites localement dans le milieu, l'intensité lumineuse.

**16.** Procédé d'optimisation de focalisation selon l'une des revendications précédentes, **caractérisé en ce que** la mesure d'intensité de la perturbation $I_m$ induite est réalisée à l'aide d'une modalité d'imagerie [8].

**17.** Procédé d'optimisation de focalisation selon la revendication 16, **caractérisé en ce que** la modalité d'imagerie [8] est choisie parmi les modalités d'imagerie à résonance magnétique, les modalités d'imagerie ultrasonore, les modalités d'imagerie tomographique par rayons x et imagerie optique.

**18.** Procédé d'optimisation de focalisation selon l'une des revendications 16 et 17, **caractérisé en ce que** la mesure d'intensité de la perturbation $I_m$ induite dans la zone d'intérêt [6] est réalisée par imagerie de la zone d'intérêt [6] elle-même.

**19.** Procédé d'optimisation de focalisation selon l'une des revendications précédentes, **caractérisé en ce que** la perturbation $I_m$ induite et mesurée dans le milieu est due à une force de radiation ultrasonore, sonore ou électromagnétique.

**20.** Système émetteur [1] comprenant un réseau de N sources [3] aptes à émettre des ondes focalisées $A_n.e^{j\alpha_n}$ présentant une distribution spatiale de phase $\alpha_n$ et d'amplitude $A_n$ ($1 \leq n \leq N$) vers une zone d'intérêt [6] d'un milieu [4], le système [1] mettant en oeuvre un procédé d'optimisation de la focalisation dans le milieu [4] selon l'une des revendications 1 à 19 utile lorsque les ondes focalisées sont émises au travers d'un élément introducteur d'aberrations [5] introduisant un décalage de phase initialement indéterminé, le système [1] comprenant pour cela des moyens ou disposant pour cela de moyens pour, lors de l'émission d'ondes focalisées dans le milieu [4],

b) modifier M-1 fois la distribution spatiale de phase $\alpha_n$ ou de la distribution d'amplitude $A_n$ ou de la distribution de phase et d'amplitude ($1 \leq n \leq N$) des ondes émises simultanément par une pluralité de sources [3] du réseau, chaque modification correspondant à des modifications simultanées des phases ou amplitudes ou phases et amplitudes sur cette pluralité de sources [3] du réseau et engendrant une émission d'ondes focalisées dans le milieu,

c) mesurer l'intensité d'une perturbation $I_m$ ($1 \leq m \leq M$) induite par les ondes dans la zone d'intérêt [6] à chaque modification m de la distribution des phases $\alpha_n$ ou de la distribution d'amplitudes $A_n$ ou de la distribution de phase et d'amplitude,

d) déduire, à partir des mesures d'intensité de perturbation $I_m$, une distribution optimale de phase d'émission $\alpha_{n_{opt}}$ ou une distribution optimale d'amplitude $A_{n_{opt}}$ ou une distribution optimale de phase et d'amplitude maximisant l'intensité de la perturbation induite dans la zone d'intérêt [6].

**21.** Système selon la revendication 20, **caractérisé en ce que** les sources [3] sont des émetteurs ultrasonores.

**22.** Produit programme d'ordinateur comportant des instructions pour l'exécution des étapes du procédé d'optimisation selon l'une quelconque des revendications 1 à 18 lorsque ledit programme est exécuté par un ordinateur.

**23.** Support d'enregistrement lisible par un ordinateur sur lequel est enregistré un programme d'ordinateur comprenant des instructions pour l'exécution des étapes du procédé d'optimisation selon l'une quelconque des revendications 1 à 19.

**Claims**

**1.** A method for optimizing the focusing of waves in an area of interest [6] of a medium [4], the waves being emitted by an array of N sources [3] towards the medium [4] through an element introducing aberrations [5] introducing an initially undetermined phase shift $\varphi_n$ ($1 \leq n \leq N$), the method using M-1 successive modifications of the emitted wave each causing a perturbation, the M perturbations are measured, these measurements being used for inferring optimum focusing characteristics, comprising the following steps:

a) emitting with the array of N sources [3], $A_n e^{j\alpha_n}$ waves (with $j^2 = -1$) having a spatial distribution of phase $\alpha_n$ and of amplitude An ($1 \leq n \leq N$) and propagating towards the area of interest [6] in the medium [4],

b) M-1 (M>1) modifications of the spatial phase $\alpha_n$ ($1 \leq n \leq N$) distribution or M-1 (M>1) modifications of the spatial amplitude $A_n$ ($1 \leq n \leq N$) distribution or M-1 (M>1) modifications of the spatial phase and amplitude (1

$\leq$ n $\leq$ N)distribution of the waves simultaneously emitted by a plurality of sources [3] of the array, each modification corresponding to simultaneous modifications of the phases and/or amplitudes on this plurality of sources [3] of the array and each generating an emission step a),

c) measuring the intensity of at least one perturbation $I_m$ ($1 \leq m \leq M$) induced by the waves in the area of interest [6] at each modification m ($1 \leq m \leq M$) of the distribution of the phases $\alpha_n$ or at each modification m ($1 \leq m \leq M$) of the distribution of amplitudes An or at each modification m ($1 \leq m \leq M$) of the phase and amplitude distribution,

d) inferring from the measurements of perturbation intensity $I_m$, an optimum emission phase $\alpha_{n_{opt}}$ distribution or an optimum amplitude $A_{n_{opt}}$ distribution or an optimum phase and amplitude distribution maximizing the intensity of the perturbation $I_m$ induced in the area of interest [6].

2. The method according to claim 1, **characterized in that** it comprises a step c') for measuring at least one perturbation $I_{m_e}$ in a point located outside the area of interest [6] at each modification m ($1 \leq m \leq M$) of the distribution of the phases $\alpha_n$ and/or of amplitudes $A_n$, step d) then being a step for inferring from the measurements of perturbation intensity $I_m$ and from the measurements of perturbation intensity $I_{m_e}$ an optimum emission phase $\alpha_{n_{opt}}$ distribution or an optimum amplitude $A_{n_{opt}}$ distribution or an optimum emission phase $\alpha_{n_{opt}}$ and amplitude $\alpha_{n_{opt}}$ distribution maximizing the intensity of the perturbation $I_m$ induced in the area of interest [6] and minimizing the intensity of the perturbation(s) $I_{m_e}$ outside the area of interest.

3. The method according to claim 1, **characterized in that** the spatial phase $\alpha_n$ ($1 \leq n \leq N$) distribution is modified by successive iterations of the phases applied on at least a plurality of sources [3], the optimum phase $\alpha_{n_{opt}}$ distribution being selected as the iteration which generates the maximum perturbation intensity.

4. The method according to claim 3, **characterized in that** the phase $\alpha_n$ distribution having been broken down into a base of K vectors $V_k$ ( $1 \leq k \leq K$), with $\alpha_n = \sum_{k=1}^{K} d_k V_{kn}$ wherein $d_k$ is a real number, each particular vector $V_k$ describing the phases emitted by the N sources [3], the phase $\alpha_n$ ($1 \leq n \leq N$) modifications are made on the set of N sources by $S_k$ modifications of the value of $d_k$ for each vector $V_k$, M then being equal to $\sum_{k=0}^{K} S_k$ .

5. The method according to claim 4, **characterized in that** the modifications of the phase $\alpha_n$ distribution are made in a subspace of K' (K'<K) vectors of the base $V_k$ with $\alpha_n = \sum_{k=1}^{K'} d_k V_{kn}$ , M then being equal to $\sum_{k=0}^{K'} S_k$ .

6. The method according to one of claims 4 and 5, **characterized in that** the vectors $V_k$ are defined from K periodic spatial functions, each coefficient $V_{kn}$ being determined by the value of a function k ($1 \leq k \leq K$) relative to the position of the source n.

7. The method according to one of claims 3 to 6, **characterized in that** the amplitude $A_n$ ($1 \leq n \leq N$) distribution is modified by successive modifications on at least one plurality of sources [3], the optimum amplitude $A_{n_{opt}}$ distribution being selected from the iteration which generates the maximum perturbation.

8. The method according to claim 7, **characterized in that** the modifications of the amplitude distribution are made successively, alternately, or simultaneously with the modification of the phase distribution.

9. The method according to claim 1, **characterized in that** the modifications of the amplitudes An and of the phases $\alpha_n$ are a combination of P vectors Hp defined in matrix form $H_{pn}$ ($1 \leq p \leq P$ and $1 \leq n \leq N$) with $A_n \cdot e^{j\alpha_n} = \sum_{p=1}^{P} c_p H_{pn}$ , wherein each value $c_p$ is a complex number.

10. The method according to claim 9, **characterized in that** the matrix $H_{pn}$ is an orthogonal matrix.

11. The method according to claim 10, **characterized in that** the matrix $H_{pn}$ is a Hadamard matrix.

12. The method according to one of claims 9 to 11, **characterized in that** the M modifications of the phase distribution or the amplitude distribution or of the phase and amplitude distribution are made by $R_p \geq 2$ modifications of the phase value $x_r$, during the emission of the sum $H_{p_1 n} + H_{pn} \cdot e^{jx_r}$, on each source n corresponding to the sum of at least two vectors $H_{p_1}$ and $H_p$ ($1 \leq p \leq P$) of the base, one of which is shifted by a phase $x_r$, M then being equal to $\sum_{p=1}^{P} R_p$, the distribution of optimum phases and amplitudes being determined by measuring the intensity of the perturbations $I_m$ obtained for each emitted sum of vectors and by inversion of the matrix $H_{pn}$.

13. The focusing optimization method according to one of the preceding claims, **characterized in that** the emitted waves are acoustic, ultrasonic, electromagnetic or optical waves.

14. The focusing optimization method according to one of the preceding claims, **characterized in that** the focusing medium [4] is a biological medium.

15. The focusing optimization method according to one of the preceding claims, **characterized in that** the perturbation $I_m$ induced and measured in the medium is selected from local displacements, local velocities, stresses, locally induced temperature variations in the medium, light intensity.

16. The focusing optimization method according to one of the preceding claims, **characterized in that** the measurement of the intensity of the induced perturbation $I_m$ is conducted by means of an imaging method [8].

17. The focusing optimization method according to claim 16, **characterized in that** the imaging method [8] is selected from magnetic resonance imaging methods, ultrasonic imaging methods, X-ray tomography imaging and optical imaging methods.

18. The focusing optimization method according to one of claims 16 and 17, **characterized in that** the intensity measurement of the perturbation $I_m$ induced in the area of interest [6] is conducted by imaging of the area of interest [6] itself.

19. The focusing optimization method according to one of the preceding claims, **characterized in that** the induced and measured perturbation $I_m$ in the medium is due to an ultrasonic, sonic or electromagnetic radiation force.

20. An emitter system [1] comprising an array of N sources [3] capable of emitting focused waves $A_n e^{j\alpha_n}$ having a spatial phase $\alpha_n$ and amplitude An ($1 \leq n \leq N$) distribution, towards an area of interest [6] of a medium [4], the system [1] applying a method for optimizing focusing in the medium [4] according to one of claims 1 to 19, useful when the focused waves are emitted through an element [5] introducing aberrations, introducing an initially undetermined phase shift, the system [1] comprising means for this or having means for this during the emission of focused waves in the medium [4], for,

   b) modifying M-1 times the spatial phase $\alpha_n$ distribution or amplitude $A_n$ distribution or phase and amplitude distribution ($1 \leq n \leq N$) of the waves simultaneously emitted by a plurality of sources [3] of the array, each modification corresponding to simultaneous modifications of the phases or of the amplitudes or of the phases and amplitudes on this plurality of sources [3] of the array and generating an emission of focused waves in the medium,
   c) measuring the intensity of a perturbation $I_m$ ($1 \leq m \leq M$) induced by the waves in the area of interest [6] at each modification m of the distribution of the phases $\alpha_n$ or of the distribution of the amplitudes $A_n$ or of the phase and amplitude distribution,
   d) inferring, from perturbation intensity $I_m$ measurements, an optimum emission phase $\alpha_{n_{opt}}$ distribution or an optimum amplitude $A_{n_{opt}}$ distribution or an optimum phase and amplitude distribution maximizing the intensity of the perturbation induced in the area of interest [6].

21. The system according to claim 20, **characterized in that** the sources [3] are ultrasonic emitters.

**22.** A computer program product including instructions for executing the steps of the optimization method according to any of claims 1 to 18, when said program is executed by a computer.

**23.** A computer-legible recording medium on which is recorded a computer program comprising instructions for executing the steps of the optimization method according to any of claims 1 to 19.

**Patentansprüche**

**1.** Verfahren zur Optimierung der Fokussierung von Wellen in einem interessierenden Bereich [6] eines Mediums [4], wobei die Wellen von einem Netz aus N Quellen [3] durch ein Aberrationen einbringendes Element [5] hindurch, das eine anfangs unbestimmte Phasenverschiebung $\varphi_n$ (1≤n≤N) einleitet, in Richtung des Mediums [4] gesandt werden, wobei das Verfahren M-1 aufeinanderfolgende Modifikationen der ausgesandten Welle, die jeweils zu einer Störung führen, verwendet, die M Störungen werden gemessen, wobei diese Messungen verwendet werden, um Merkmale einer optimalen Fokussierung abzuleiten, umfassend die folgenden Schritte:

a) Aussenden, durch das Netz aus N Quellen [3], von Wellen $A_n . e^{j\alpha_n}$ (mit j² = -1), die eine räumliche Phasenverteilung $\alpha_n$ und Amplitudenverteilung An (1≤n≤N) aufweisen und sich in Richtung des interessierenden Bereichs [6] in dem Medium [4] fortpflanzen,

b) M-1 (M>1) Modifikation(en) der räumlichen Phasenverteilung $\alpha_n$ (1≤n≤N) oder M-1 (M>1) Modifikation(en) der räumlichen Amplitudenverteilung An (1≤n≤N) oder M-1 (M>1) Modifikation(en) der räumlichen Phasen- und Amplitudenverteilung (1≤n≤N) der durch eine Vielzahl von Quellen [3] des Netzes gleichzeitig ausgesandten Wellen, wobei jede Modifikation gleichzeitigen Modifikationen der Phasen und/oder Amplituden an dieser Vielzahl von Quellen [3] des Netzes entspricht und jeweils einen Sendeschritt a) erzeugt,

c) Messen der Intensität wenigstens einer durch die Wellen in dem interessierenden Bereich [6] induzierten Störung $I_m$ (1≤m≤M) bei jeder Modifikation m (1≤m≤M) der Verteilung der Phasen $\alpha_n$ oder bei jeder Modifikation m (1≤m≤M) der Amplitudenverteilung $A_n$ oder bei jeder Modifikation m (1≤m≤M) der Phasen- und Amplitudenverteilung,

d) Ableiten, aus den Messungen der Intensität einer Störung $I_m$, einer optimalen Emissionsphasenverteilung $\alpha_{n_{opt}}$ oder einer optimalen Amplitudenverteilung $A_{n_{opt}}$ oder einer optimalen Phasen- und Amplitudenverteilung, die die Intensität der in dem interessierenden Bereich [6] induzierten Störung $I_m$ maximiert.

**2.** Verfahren nach Anspruch 1, **dadurch gekennzeichnet, daß** es einen Schritt c') zur Messung wenigstens einer Störung $I_{m_e}$ an einer außerhalb des interessierenden Bereichs [6] gelegenen Stelle bei jeder Modifikation m (1≤m≤M) der Verteilung der Phasen $\alpha_n$ und/oder Amplituden $A_n$ umfaßt, wobei der Schritt d) dann ein Schritt zum Ableiten einer optimalen Emissionsphasenverteilung $a_{n_{opt}}$ oder einer optimalen Amplitudenverteilung $A_{n_{opt}}$ oder einer optimalen Emissionsphasen- $a_{n_{opt}}$ und Amplitudenverteilung $A_{n_{opt}}$, die die Intensität der in dem interessierenden Bereich [6] induzierten Störung $I_m$ maximiert und die Intensität der Störung oder Störungen $I_{m_e}$ außerhalb des interessierenden Bereichs minimiert, aus den Messungen der Intensität einer Störung $I_m$ und aus den Messungen der Intensität einer Störung $I_{m_e}$ ist.

**3.** Verfahren nach Anspruch 1, **dadurch gekennzeichnet, daß** die räumliche Phasenverteilung $\alpha_n$ (1≤n≤N) durch sukzessive Iterationen der Phasen, die auf wenigstens eine Vielzahl von Quellen [3] angewandt werden, modifiziert wird, wobei die optimale Phasenverteilung $a_{n_{opt}}$ als die Iteration gewählt wird, die die maximale Störungsintensität erzeugt.

**4.** Verfahren nach Anspruch 3, **dadurch gekennzeichnet, daß** - wenn die Phasenverteilung $\alpha_n$ in eine Basis von K Vektoren $V_k$ (1≤k≤K), mit $\alpha_n = \sum_{k=1}^{K} d_k V_{kn}$, worin $d_k$ eine reelle Zahl ist, wobei jeder Einzelvektor $V_k$ die durch die N Quellen [3] ausgesandten Phasen beschreibt, zerlegt wird - die Phasenmodifikationen $\alpha_n$ (1≤n≤N) an allen N Quellen durch $S_k$ Modifikationen des Wertes von $d_k$ für jeden Vektor $V_k$, wobei M dann gleich $\sum_{k=0}^{K} S_k$ ist, durchgeführt werden.

5. Verfahren nach Anspruch 4, **dadurch gekennzeichnet, daß** die Modifikationen der Phasenverteilung $\alpha_n$ in einem Unterraum von K' (K'<K) Vektoren der Basis $V_k$, mit $a_n = \sum_{k=1}^{K'} d_k V_{kn}$, wobei M dann gleich $\sum_{k=0}^{K'} S_k$ ist, durchgeführt werden.

6. Verfahren nach einem der Ansprüche 4 und 5, **dadurch gekennzeichnet, daß** die Vektoren $V_k$ anhand von K periodischen Funktionen des Raums definiert werden, wobei jeder Koeffizient $V_{kn}$ durch den Wert einer Funktion k (1≤k≤K) bezüglich der Position der Quelle n bestimmt wird.

7. Verfahren nach einem der Ansprüche 3 bis 6, **dadurch gekennzeichnet, daß** die räumliche Amplitudenverteilung $A_n$ (1≤n≤N) durch sukzessive Modifikationen an wenigstens einer Vielzahl von Quellen [3] modifiziert wird, wobei die optimale Amplitudenverteilung $\alpha_{n_{opt}}$ als die Iteration gewählt wird, die die maximale Störung erzeugt.

8. Verfahren nach Anspruch 7, **dadurch gekennzeichnet, daß** die Modifikationen der Amplitudenverteilung sukzessive, abwechselnd oder gleichzeitig mit der Modifikation der Phasenverteilung durchgeführt werden.

9. Verfahren nach Anspruch 1, **dadurch gekennzeichnet, daß** die Modifikationen der Amplituden $A_n$ und der Phasen $\alpha_n$ eine Kombination aus P Vektoren H p sind, die in einer Matrixform $H_{pn}$ (1≤p≤P und 1≤n≤N), mit

$$A_n . e^{j\alpha_n} = \sum_{p=1}^{P} c_p H_{pn}$$, worin jeder Vektor $c_p$ eine komplexe Zahl ist, definiert sind.

10. Verfahren nach Anspruch 9, **dadurch gekennzeichnet, daß** die Matrix $H_{pn}$ orthogonal ist.

11. Verfahren nach Anspruch 10, **dadurch gekennzeichnet, daß** die Matrix $H_{pn}$ eine Hadamard-Matrix ist.

12. Verfahren nach einem der Ansprüche 9 bis 11, **dadurch gekennzeichnet, daß** die M Modifikationen der Phasenverteilung oder der Amplitudenverteilung oder der Phasen- und Amplitudenverteilung durch $R_p \geq 2$ Modifikationen des Phasenwertes $x_r$ bei Senden der Summe H $_{pln}$ + H pn.$e^{jx_r}$ an jeder Quelle n, die der Summe aus wenigstens zwei Vektoren H pl und H p (1≤p≤P) der Basis entspricht, von denen einer um eine Phase $x_r$ verschoben ist, durchgeführt werden, wobei M dann gleich $\sum_{p=1}^{P} R_P$ ist, wobei die optimale Phasen- und Amplitudenverteilung mit Hilfe der Messung der Intensität der für jede gesandte Vektorensumme erhaltenen Störungen $l_m$ sowie durch Inversion der Matrix H $_{pn}$ bestimmt wird.

13. Verfahren zur Optimierung der Fokussierung nach einem der vorhergehenden Ansprüche, **dadurch gekennzeichnet, daß** die ausgesandten Wellen akustische, Ultraschall-, elektromagnetische oder optische Wellen sind.

14. Verfahren zur Optimierung der Fokussierung nach einem der vorhergehenden Ansprüche, **dadurch gekennzeichnet, daß** das Fokussierungsmedium [4] ein biologisches Medium ist.

15. Verfahren zur Optimierung der Fokussierung nach einem der vorhergehenden Ansprüche, **dadurch gekennzeichnet, daß** die in dem Medium induzierte und gemessene Störung $l_m$ aus lokalen Bewegungen, lokalen Geschwindigkeiten, Spannungen, in dem Medium lokal induzierten Temperaturschwankungen, Lichtstärke ausgewählt ist.

16. Verfahren zur Optimierung der Fokussierung nach einem der vorhergehenden Ansprüche, **dadurch gekennzeichnet, daß** die Messung der Intensität der induzierten Störung $l_m$ mit Hilfe einer Bildgebungsmodalität [8] durchgeführt wird.

17. Verfahren zur Optimierung der Fokussierung nach Anspruch 16, **dadurch gekennzeichnet, daß** die Bildgebungsmodalität [8] aus Magnetresonanz-Bildgebungsmodalitäten, Ultraschall-Bildgebungsmodalitäten, Röntgentomographie- und optischen Bildgebungsmodalitäten ausgewählt ist.

**18.** Verfahren zur Optimierung der Fokussierung nach einem der Ansprüche 16 und 17, **dadurch gekennzeichnet, daß** die Messung der Intensität der in dem interessierenden Bereich [6] induzierten Störung $I_m$ mittels Bildgebung des interessierenden Bereichs [6] selbst durchgeführt wird.

**19.** Verfahren zur Optimierung der Fokussierung nach einem der vorhergehenden Ansprüche, **dadurch gekennzeichnet, daß** die in dem Medium induzierte und gemessene Störung $I_m$ durch eine Ultraschall-, Schall- oder elektromagnetische Strahlungskraft bedingt ist.

**20.** Sendesystem [1], umfassend ein Netz aus N Quellen [3], die geeignet sind, fokussierte Wellen $A_n.e^{j\alpha_n}$ mit einer räumlichen Phasenverteilung $\alpha_n$ und Amplitudenverteilung $A_n$ ($1\leq n\leq N$) in Richtung eines interessierenden Bereichs [6] eines Mediums [4] auszusenden, wobei das System [1] ein Verfahren zur Optimierung der Fokussierung in dem Medium [4] nach einem der Ansprüche 1 bis 19 verwendet, das nützlich ist, wenn die fokussierten Wellen durch ein Aberrationen einbringendes Element [5] hindurch, das eine anfangs unbestimmte Phasenverschiebung einleitet, gesandt werden, wobei das System [1] hierfür Mittel umfaßt oder hierfür über Mittel verfügt, um beim Aussenden von fokussierten Wellen in das Medium [4]

b) die räumliche Phasenverteilung $\alpha_n$ oder die Amplitudenverteilung $A_n$ oder die Phasen- und Amplitudenverteilung ($1\leq n\leq N$) der durch eine Vielzahl von Quellen [3] des Netzes gleichzeitig ausgesandten Wellen M-1 mal zu modifizieren, wobei jede Modifikation gleichzeitigen Modifikationen der Phasen oder Amplituden oder Phasen und Amplituden an dieser Vielzahl von Quellen [3] des Netzes entspricht und ein Aussenden von fokussierten Wellen in das Medium erzeugt,
c) die Intensität einer durch die Wellen in dem interessierenden Bereich [6] induzierten Störung $I_m$ ($1\leq m\leq M$) bei jeder Modifikation m der Verteilung der Phasen $\alpha_n$ oder der Amplitudenverteilung $A_n$ oder der Phasen- und Amplitudenverteilung zu messen,
d) aus den Messungen der Intensität einer Störung $I_m$ eine optimale Emissionsphasenverteilung $a_{n_{opt}}$ oder eine optimale Amplitudenverteilung $a_{n_{opt}}$ oder eine optimale Phasen- und Amplitudenverteilung, die die Intensität der in dem interessierenden Bereich [6] induzierten Störung maximiert, abzuleiten.

**21.** System nach Anspruch 20, **dadurch gekennzeichnet, daß** die Quellen [3] Ultraschallsender sind.

**22.** Computerprogrammprodukt, umfassend Befehle für die Durchführung der Schritte des Optimierungsverfahrens nach einem der Ansprüche 1 bis 18, wenn das Programm durch einen Computer ausgeführt wird.

**23.** Computerlesbarer Aufzeichnungsträger, auf dem ein Computerprogramm gespeichert ist, das Befehle für die Durchführung der Schritte des Optimierungsverfahrens nach einem der Ansprüche 1 bis 19 umfaßt.

FIG.1

FIG.2

FIG.3

FIG.4a

FIG.4b

FIG.4c

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2843874
- US 2004012323 A
- US 5590657 A

**Littérature non-brevet citée dans la description**

- **SINKUS R ; TANTER M ; XYDEAS T et al.** *MAGNETIC RESONANCE IMAGING,* vol. 23 (2), 159-165